Europäisches Patentamt

European Patent Office

Office européen des brevets

(19)

(11) Publication number: **0 383 090**

**A1**

(12) # EUROPEAN PATENT APPLICATION

(21) Application number: **90101804.4**

(51) Int. Cl.5: **C12P 21/00, C12N 5/00, A61K 39/40**

(22) Date of filing: **30.01.90**

The title of the invention has been amended (Guidelines for Examination in the EPO, A-III, 7.3).

(30) Priority: **30.01.89 JP 22245/89**
**17.10.89 JP 271034/89**

(43) Date of publication of application:
**22.08.90 Bulletin 90/34**

(84) Designated Contracting States:
**AT BE CH DE FR GB IT LI NL SE**

(71) Applicant: **SUMITOMO CHEMICAL COMPANY, LIMITED**
**5-33, Kitahama 4-chome Chuo-ku**
**Osaka(JP)**

Applicant: **SUMITOMO PHARMACEUTICALS COMPANY, LIMITED**
**2-8, Doshomachi 2-chome, Chuo-ku**
**Osaka-shi Osaka 541(JP)**

(72) Inventor: **Ochi, Hiroshi**
**2-11-8-110, Sonehigashi-machi**
**Toyonaka, Osaka(JP)**
Inventor: **Ohtsuka, Hiroshi**
**16-40-601, Takagihigashi-machi**
**Mishinomiya, Hyogo(JP)**
Inventor: **Yokota, Shinichi**
**2-14-7, Mefu**
**Takarazuka, Hyogo(JP)**
Inventor: **Noguchi, Hiroshi**
**4-4-153 Seiwadainishi**
**Kawanishi, Hyogo(JP)**
Inventor: **Terashima, Masazumi**
**2-1-125, Kuwata-cho**
**Ibaraki, Osaka(JP)**
Inventor: **Kato, Masuhiro**
**1-13-1-603, Arima, Miyamae-ku**
**Kawasaki, Kanagawa(JP)**

(74) Representative: **Kolb, Helga, Dr. Dipl.-Chem. et al**
**Hoffmann, Eitle & Partner Patentanwälte**
**Arabellastrasse 4**
**D-8000 München 81(DE)**

(54) **Human monoclonal antibody to pseudomonas Aeruginosa, and its production and use.**

(57) A human monoclonal antibody showing a specific binding property to flagella of Pseudomonas aeruginosa, characterized in that said antibody produces a therapeutic effect on the mouse experimental infection caused by Pseudomonas aeruginosa at a dose of not less than 5 μg/kg of body weight.

## HUMAN MONOCLONAL ANTIBODY, AND ITS PRODUCTION AND USE

The present invention relates to a human monoclonal antibody to Pseudomonas aeruginosa (hereinafter referred to as "P. aeruginosa"), and its production and use. More particularly, it relates to a human monoclonal antibody, which has a specific binding property to flagella of P. aeruginosa, and its production and use.

Noticeably, the human monoclonal antibody shows its binding property not only to various strains of different serotypes based on O-antigen but also to strains belonging to the serotype having no O-antigen, i.e. Type M according to the classification by Nippon Ryokunoh-kin Kenkyukai Kesseigata-betsu Kento linkai (Commitee of Study on Serotype Classification, Japanese Study Group on Pseudomonas aeruginosa) (hereinafter referred to as "Japanese Committee"). Further, it exerts a remarkable therapeutic effect on the experimental mouse infection caused by P. aeruginosa at a dose of not less than 5 μg/kg of body weight. Thus, the human monoclonal antibody is useful as a prophylactic or therapeutic agent on infectious diseases caused by P. aeruginosa and also as a diagnostic agent for such infectious diseases.

The kinds of bacteria causing infectious diseases, i.e. .infection-causing bacteria, have changed with development of antibiotics as clinically used. As the result, infections with bacteria originally having only low pathogenicity or virulence have increased, and P. aeruginosa is currently one of the major pathogenic bacteria causing infectious diseases, of which serious symptoms often lead patients to death, particularly when their immunological activity is low due to continuous administration of immunosupressants or they are suffered from immunodeficiency or immunodepression diseases caused by cancer, burn or the like.

Among various prophylactic or therapeutic methods for bacterial infections, the most prevailing one is chemotherapy by the use of antibiotics or antimicrobial agents. In fact, there have been developed various antibiotics including streptomycin, kanamycin, penicillin, cephalosporin, etc., which are generally sensitive to Gram-positive bacteria (e.g. Staphylococci) and Gram-negative bacteria (e.g. E. coli) and produce a prominent clinical effect. However, there are known only few medicinal products sensitive to P. aeruginosa, and even those medicinal products act on P. aeruginosa only bacteriostatically but not bacteriocidally. Thus, they can prevent the growth of P. aeruginosa but do not exert any remarkable therapeutic effect in clinic.

The other prophylactic or therapeutic method is antibody therapy by administration of immunoglobulin. This method is performed in place of or sometimes in association with chemotherapy. A serum of high antibody titer can be obtained by active immunization of animals such as horse or rabbit, and antibody therapy can be made through administration of such serum. In fact, its remarkable therapeutic effect has been proven on experimental infections using various animals. As seen in the cases of diphtheria toxin and viper toxin, antibody therapy using sera originated from animals is quite effective even for human beings. However, introduction of a heterogenic protein obtained from animals into a human body causes such a serious side-effect as anaphylaxis or any other allergic reaction, so that the sera originated from animals is not used therapeutically for bacterial infections. It is thus highly desired to develop human immunoglobulin having a high antibody titer against bacteria and showing a prominent therapeutic effect on bacterial infections.

Conventional human immunoglobulin preparations are manufactured by collecting blood from healthy persons or bacteria-infected patients, subjecting the blood to fractionation to obtain an immunoglobulin fraction, purifying the immunoglobulin fraction and eliminating therefrom agglutinated materials through addition of ethylene glycol, treatment with protease, sulfonization, DEAE-column chromatography, etc., followed by formulation of the resulting product into intramuscularly or intravenously injectionable preparations. These preparations are advantageous in not causing anaphylaxis or any other side-effect as seen in administration of immunoglobulin originated from animals but yet have some drawbacks. One of such drawbacks is that their antibody titer against bacteria is low, so that a sufficient therapeutic effect can not necessarily be produced. Another drawback is that their stable supply with a high antibody titer in a large amount is difficult, because they are prepared with the blood collected from healthy persons or bacteria-infected patients. So, the constant and continuous obtainment of sera having a high antibody titer is quite hard. A further drawback is that they may be contaminated with hepatitis virus (e.g. HB virus), Adult T cell leukaemia virus (ATLV, HTLV), etc., since the blood as the starting material is obtained from a number of persons. In order to avoid these drawbacks, production of a human monoclonal antibody having a strong prophylactic or therapeutic effect on bacterial infections caused by P. aeruginosa is highly demanded.

On the surface layer of the bacterial body of P. aeruginosa, there exist a large number of antigens, on which many studies and researches have targetted so as to provide a vaccine or antibody to be prophylactically or therapeutically usable in treatment of infectious diseases caused by P. aeruginosa. As the target antigen, there are known O-antigens, outer membrane proteins, cilia (i.e. pili or fimbriae), flagella, mucoids, etc.

The O-antigens are widely used for classification of P. aeruginosa strains on their serotypes and correspond mainly to differences in the construction (i.e. component, sequence and configuration) of the repeating unit of the O-polysaccharide portion in the lipopolysaccharide (LPS) existing in the outer membrane. Typical examples of the serotype classification are as follows: Types 1 to 17 according to the classification by Homma et al. (Japan. J. Exp. Med., 44, 1, (1974)); Types 1 to 7 according to the classification by Fisher et al (J. Bacteriol., 98, 835 (1969)); Types A to M according to the classification by Japanese Committee (Japan. J. Exp. Med., 45, 329 (1976)); Types 1 to 17 according to the classification by International Antigenic Typing System (IATS) (Int. J. Syst. Bacteriol., 33, 256 (1983)), etc. These classifications and their relationship are shown in Table 1 (Japan. J. Exp. Med., 46, 329 (1976)).

Table 1:

| Serotype classification of P . aeruginosa | | | |
|---|---|---|---|
| Japanese Commitee 1976 | Homma et al 1974 | ITATS 1983 | Fisher et al 1969 |
| A | 1 | 3 | - |
| B | 2, 7, 13, 16 | 2, 5, 16 | 3, 7 |
| C | 3 | 8 | 6 |
| D | 4 | 9 | - |
| E | 5 | 11 | 2 |
| F | 6 | 4 | - |
| G | 8 | 6 | 1 |
| H | 9 | 10 | 5 |
| I | 10 | 1 | 4 |
| J | 11 | 15 | - |
| K | 12 | 13 | - |
| L | 14 | - | - |
| M | 15, 17 | - | - |
| - | - | 7, 12, 14, 17 | - |

It is known that the antibody specific to a certain O-antigen shows a strong prophylactic or therapeutic effect against P. aeruginosa strains of the serotype to which said O-antigen belongs but not against P. aeruginosa strains of other serotypes. Further, even the antibody effective to the O-antigen in any serotype is not effective to Type M strains which do not possess any O-antigen. In order for the antibody to be effective against P. aeruginosa strains of every serotype, it may be considered to use all the monoclonal antibodies corresponding to those serotypes in mixture. This technique is, however, extremely troublesome and so far hardly applicable to the practical use.

Major outer membrane proteins are antigens common to P. aeruginosa strains. For instance, Hancock et al. confirmed eight proteins (D1, D2, E, F, G, H1, H2 and I) as the outer membrane protein and disclosed a part of their characteristics (J. Bacteriology, 140, 802 (1979)). Further investigation using anti-serums against the outer membrane protein revealed that the outer membrane proteins designated as F, H2 and I could possibly be used as a vaccine (J. Infec. Disease, 146, 770 (1982)). Based on these knowledge, they succeeded in obtaining a mouse monoclonal antibody to H2 and I proteins (Infect. Immun., 37, 166 (1982)) as well as a mouse monoclonal antibody to F protein (Infec. Immun., 42, 1027 (1983)) and subsequently confirmed that the mouse monoclonal antibody corresponding to F protein is effective in treatment of the in vivo experimental P. aeruginosa infection in mice (Eur. J. Clin. Microbiol., 4, 224 (1985)). Likewise, Sokol et al. reported that ferripyochelin-binding protein (FBP) is an antigen common to P. aeruginosa strains and a mouse monoclonal antibody to said FBP is effective in treatment of the experimental P. aeruginosa infection in mice (Infect. Immun., 51, 896 (1986)). Nevertheless, current studies and researches on the outer membrane proteins have not sufficiently been developed, and un clarification still remains on the characteristics, functions, etc. of each outer membrane protein of P. aeruginosa, particularly on the relationship of such outer membrance protein with the pathogenicity of P. aeruginosa.

Cilia, flagella and mucoids existing on the outside of the outer membrane are known to have a close correlation with the pathogenicity of P. aeruginosa. Woods et al., for instance, reported that cilia take part in the adherence of P. aeruginosa onto the upper respiratory epithelium, followed by colonization (Infect. Immun., 29, 1146 (1980)). Also, Ramphal et al. confirmed that said adherence has a correlation to cilia and mucoids (Infect. Immun., 44, 38 (1984); ibid., 47, 1 (1985)). Further, McManus et al. (Burns, 6, 135, 1980) and Carven et al. (Can. J. Microbiol., 25, 458 (1981)) reported that a motility of P. aeruginosa due to flagella is correlated to its pathogenicity. Furthermore, Montie et al. made comparison of toxicities in the experimental infections of burned mice with P. aeruginosa strains having flagella and with their mutated strains having no flagella and confirmed that flagella is the important pathogenic factor (Infect. Immun., 38, 1296 (1982)). Moreover, Montie et al. reported that in the in vitro experiment the anti-serum to flagella could suppress the motility of P. aeruginosa (Infect. Immun., 35, 182 (1982)).

In addition, Holder et al. reported the effectiveness of the preparation originated from flagella as a vaccine in the experimental P. aeruginosa infections with burned mice (infect. Immun., 35, 220 (1982)). Drake et al. showed the effectiveness of the anti-serum to flagella in the experimental P. aeruginosa infections with burned mice (Can. J. Microbiol,, 33, 755-763 (1987)). Lostrom et al. provided a hybridoma cell line capable of producing a mouse monoclonal antibody to flagella of P. aeruginosa as well as an EB virus transformed cell line capable of producing a human monoclonal antibody to flagella of P. aeruginosa and submitted an experimental report on the therapeutic effect of those antibodies in the experimental P. aeruginosa infections in mice (JP-A-63-102697). From their experimental report, however, it is noted that the therapeutic effect is obtainable only at a relatively high dose. Namely, in case of the subcutaneous administration of the human monoclonal antibody premixed with bacteria, the dose of said antibody is to be 10 μg per animal for exertion of its therapeutic effect, and in case of the intravenous administration of the human monoclonal antibody 2 hours before the bacterial infection, the does is to be 40 μg (corresponding to 0.5 - 2 mg/kg) (of. Examples 8, 10, 11 and 12 in JP-A-63-102697). Based on these experiments, the possible effective dose for human beings would be about 1 to 200 mg/kg for the therapeutic purpose and about 0.1 to 25 mg/kg for the preventive purpose. Taking the currently available technique for cultivation of the animal cells and purification of the monoclonal antibody into consideration, the mass production of the prophylactic or therapeutic agent according to Lostrom et al. at a low cost will be difficult. In order to overcome this difficulty, it will be necessary to produce a human monoclonal antibody which is highly active and therefore low in its dose.

Among various infectious diseases caused by P. aeruginosa, cystic fibrosis and diffuse parbronchiolitis (DPB) similar thereto are particularly serious and produce a high percentage of death. In these serious diseases, there are frequently isolated Type M strains possessing no O-antigen as the causative bacterium (Infec. Immun., 42, 170-177 (1983)). There has not been so far any report on the antibody exerting a therapeutic effect on Type M strains, and the establishment of the antibody effective to said strains is highly desired.

As the result of an extensive study, it has now been succeeded in providing a human monoclonal antibody which shows a specific binding property to flagella of P. aeruginosa strains, of which the serotype on O-antigen may be of anyone including Type M having no O-antigen. The human monolconal antibody exerts a noticeable therapeutic effect in the mouse experimental P. aeruginosa infectious diseases even at such a small dose as about 5 μg/kg per body weight or more.

Accordingly, a basic object of the present invention is to provide a human monoclonal antibody showing a specific binding property to flagella of P. aeruginosa strains, which are not restricted to certain definite serotypes but may be of any serotype including Type M having no O-antigen, and exerting its therapeutic effect on infectious diseases caused by P. aeruginosa at a relatively low dose such as about 5 μg/kg per body weight or more. Another object of this invention is to provide a human cell line capable of continuously producing said human monoclonal antibody and its descendant cell lines. A further object of the invention is to provide a process for production of said human monoclonal antibody by the in vitro cultivation of said human cell line. A still further object of the invention is to provide a human immunoglobulin preparation comprising said human monoclonal antibody, which is usable for prophylactic or therapeutic treatment of the infections caused by P aeruginosa.

A flagellum is a motile small organ and comprises a basal body bound on the outer surface layer of the cell body and a spiral fiber protruded therefrom with a hook connecting them. In case of P. aeruginosa, the spiral fiber is made of a kind of flagellin, of which units are spirally connected by hydrophobic bonding to form a cylindrical structure. Flagellin is a granular protein, and that of P. aeruginosa is readily obtainable by the Pitt et al. method (J. Med. Microbiol., 14, 251 (1981)) and can be purified with ease according to the affinity column chromatography using a human monoclonal antibody corresponding thereto.

As to the biochemical observation of flagella of P. aeruginosa, Montie et al. reported that the apparent molecular weight of flagellin constituting said flagella is in a range of 53,000 to 45,000 when determined by sodium dodecylsulfate-polyacrylamide gel electrophoresis (herein after referred to as "SDS-PAGE") (Infec.

Immun., 49, 770 (1985)). Further, Lanyi et al. reported that a thermolabile antigen (i.e. H-antigen), which is often detected in the agglutination method for classification of P. aeruginosa, is mainly correlated to flagella and established the classification of flagella on the serotype (Acta Microbiol. Acad. Sci. Hung., 17, 35 (1970)). Similar reports were also made by Ansorg et al. (Zentralbl. Bakteriol. Parasitenkd. Infektionskr. Hyg. Erste Abt. orig. Reihe A Med. Mikrobiol. Parasitol., 242, 228 (1978)) and by Pitt et al. (J. Med. Microbiol., 14, 251 (1981)). While the relationship of the serotypes in these classifications are not clear, Pitt et al. stated that Pitt H3 corresponds to Lanyi H1 and Ansorg Hb and is a homogeneous serotype having no subgroup, and Pitt H1, H2, H4, H5 and H6 correspond to Lanyi H2 and Ansorg Ha and is a heterogeneous serotype having a subgroup.

The term "monoclonal antibody" herein used means an antibody having a uniform molecular structure and being produced by a single antibody-producing clone, which is obtainable by cell fusion (Nature, 256, 495 (1975)), EB (Esptein-Barr) virus transformation (Proc. Natl. Acad. Sci. USA., 70 (1973)) or the like. The antibody is a glycoprotein having a specific binding property to an antigen and can be produced by B lymphocytes. It is classified into the following groups: IgG, IgM, IgD, IgA and IgE; for instance, IgG has a molecular weight of about 150,000 and comprises two heavy chains (H chains) having each a molecular weight of about 50,000 and two light chains (L chains) having each a molecular weight of about 25,000, each of said H chains and L chains being covalently bound through a disulfide residue and one molecule of IgG being bound specifically to two molecules of antigen.

Evaluation of the therapeutic effect of the human monoclonal antibody in an experimental P. aeruginosa infectious diseases in mice is made as follows: into groups of mice received a designed amount of the human monoclonal antibody (i.e. medicaged group), various amounts of P. aeruginosa cells are inoculated, and the $LD_{50}$ value (the amount of cells leading half the mice to death) and the upper and lower limits of the $LD_{50}$ value with 95 % confidence limit are statistically determined; the same determination as above is also made on the non-medicated group or the BSA (bovine serum alubmin) administration group for comparison; when the $LD_{50}$ value range of the medicated group is not overlapped with that of the group for comparison, it is taken as the presence of significant difference between them, and the human monoclonal antibody is regarded to be therapeutically effective.

The present invention will be further explained in details below.

The human monoclonal antibody of the invention is a homogeneous antibody of human type originated from a single antibody-producing cell clone, which can recognize specifically flagellin constituting the flagella of P. aeruginosa as an antigenic determinant, has a specific binding property to said flagella and produces a definite therapeutic effect in the experimental P. aeruginosa infections with burned mice by intraveous administration at a dose of 5 μg/kg of body weight or more. This antibody is quite characteristic in showing a binding property to a wide variety of P. aeruginosa strains of different serotypes based on O-antigen, particularly including Type M strains having no O-antigen.

For instance, the antibody produced from Hybridoma IN-2A8 shows a binding property to 50 to 65 % of the clinically isolated strains of P. aeruginosa belonging to Type A (classified based on O-antigen), 50 to 60 % of those in Type B, 40 to 50 % of those in Type H, about 30 % of those in Type I and about 35 % of those in Type M without any material binding property to those of Types E and G. Thus, the antibody shows a binding property to 30 % or more of all the clinically isolated strains including Types A, B, E, G, H, I and M. Due to such wide recognizability, the antibody is usable for isolation and identification of P. aeruginosa strains. In addition, the antibody specifically recognizes flagella or flagellin as its constitutent, which correspond to Type Hb by the Ansorg et al. classification.

Accordingly, the human monoclonal antibody of the invention may be used for prevention and treatment of a wide variety of infectious diseases with P. aeruginosa of different serotypes based on O-antigen, particularly including Type M strains having no O-antigen by its administration at a low concentration, which causes lysis of the bacterium by a complement, acceleration of phagocytosis of a macro phage and supression of the motility of bacterium, leading to make P. aeruginosa less virulent.

The human monoclonal antibody of the invention generally belongs to IgM but is not limited to said class. For production of such antibody, there may be adopted a method which comprises fundamentally the following stages: (1) preparation of human B lymphocytes sensitized with an antigen; (2) establishment of the cell line capable of producing a specific monoclonal antibody by immortalization of the B lymphocytes as prepared in (1); (3) cultivation of the cell line as established in (2); (4) purification of the monoclonal antibody from the culture supernatant as obtained in (3); and (5) production of an immunogloblin preparation of high titer comprising the monoclonal antibody as purified in (4). Each of these stages will be hereinafter illustrated in details.

Stage (1):-

As the human B lymphocites, there may be used human lymphocyte cells producing an antibody to flagella of P. aeruginosa, which can be separated from a peripheral blood by the centrifugation using a lymphocyte separation liquid such as Lymphoprep® or Mono-Poly Resolving Medium® (Flow Lab.). There may be also used B lymphocytes originated from tissues or organs (e.g. lymph node, spleen) extracted for the purpose of diagnosis or therapy of diseases, umbilical cord blood or the like. It is desirable to obtain the cells from persons once infected with P. aeruginosa and whose cells are sensitized by the infection. Pertinent persons, from whom the cells may be obtained, can be chosen by previous measurement of the antibody titer in their sera to formalin-fixed P. aeruginosa cells or flagella of P. aeruginosa by the ELISA method or the radioimmunoassay method. Alternatively, human B lymphocytes may be obtained from any person irrespective of his or her past medical history. Such lymphocytes are mixed with formalin-fixed P. aeruginosa cells or preferably flagella of P. aeruginosa as an antigen for in vitro sensitization. Also, solutions containing lymphokines such as B cell proliferation factors and B cell differentiation factors (e.g. plant lectins such as pokeweed mitogen (PWM), bacterial components such as Cowan I, human mixed lymphocyte culture supernatant, spleen, thymus or umbilical cord blood cell culture supernatant) may be added to human B lymphocytes culture for sensitization in vitro, followed by proliferation and differentiation to give antibody-producing cells. These procedures such as preselection and in vitro sensitization are effective to obtain the spefic antibody-producing cell lines with a high efficiency after cell fusion. The thus obtained human B lymphocytes characteristically have antibody molecules at the cell surface and can release a certain amount of antibody for a limited period of culture, but their proliferation over an unlimited period of time is not possible.

Stage (2):-

For changing the above sensitized human B lymphocytes to continuously and perpetually proliferable cell lines, the sensitized human lymphocytes are subjected to cell fusion with myeloma cells in the presence of polyethylene glycol (PEG). The myeloma cells as used are hypoxanthine-guanine-phosphoribosyl transferase (HGPRT)-deficient mutants (e.g. P3X63-Ag 8(P3), P3X63-Ag 8.653) originated from mouse myeloma cells, HGPRT-deficient mutants originated from mouse-human heteromyeloma cells obtained by cell fusion between mouse myeloma cells and human myeloma cells or between mouse myeloma cells and human lymphocyte B cells, etc.

As the polyethylene glycol (PEG), there may be used, for instance, PEG 1,000 to 6,000 in a concentration of 30 to 50 % (w/v). The fusion efficiency can be enhanced in the presence of lectin, poly-L-lysine, dimethylsulfoxide (DMSO), etc.

The fusion may be carried out, for instance, in the same manner as described in the Kohler et al. article (Nature, 256, 495 (1975)) wherein mouse cells are fused each other to obtain a hybridoma producing a mouse monoclonal antibody. For instance, antigen-sensitized human lymphocytes and HGPRT-deficient myeloma cells or human-mouse heteromyeloma cells are mixed together in a proportion of 3 - 1 : 1, 45 % (w/v) PEG 1500-6000 is added portionwise thereto in a period of 0.5 to 1 minute, and the resultant mixture is allowed to stand for 0.5 to 3 minutes. To the resulting mixture, 10 to 50 ml of a culture medium containing no serum are added in 5 to 10 minutes, and subsequently 2 ml of FCS are added, followed by incubation at 37°C for 10 to 60 minutes. After centrifugation, fresh culture medium is further added thereto to make a cell concentration of $10^5$ to $10^6$/ml. The cell suspension thus obtained is seeded to a 96 well microplate at a rate of $2 \times 10^4$ to $2 \times 10^5$ cells per well. On the next day, the half amount is replaced by a hypoxanthine-aminopterin-thymidine-containing medium (HAT medium) or a hypoxanthine-azaserine-containing medium (HAz medium), and cultivation is done at 32 to 37°C under 5 % $CO_2$. For about 10 to 20 days, the culture medium is replaced by fresh HAT medium or HAz medium, and subsequently by hypoxanthine-thymidine-containing medium (HT medium) or hypoxanthine-containing medium (H medium) for about 3 to 5 days, each replacement being made every 3 days for the half amount over a period of about 2 to 3 weeks to obtain a proliferative colony, i.e. hybridoma. It is also possible to select a hybridoma by the combined use of metabolism inhibitors without using a HGPRT-deficient mutant.

The antibody titer of the culture medium to formaline-fixed P. aeruginosa cells or to flagella of P. aeruginosa is determined by ELISA or radioimmunoassay (RIA), if necessary, in combination with Western blotting to select the desired cell capable of producing the specific antibody to flagellin of P. aeruginosa. Cloning is repeated two or three times by the limiting dilution method or the agarose method to obtain a stable cell line having a high proliferation rate and a high specific antibody productivity.

The cell lines as established from sensitized human B lymphocytes according to the cell fusion (hybridoma) method can be proliferated continuously, and in addition, they can be cultivated in a medium containing no serum at a large scale to give stably a large amount of the specific antibody.

Stage (3):-

The thus established hybridomas (0.5 - 5 x $10^5$ cells/ml) are cultured in a stationary or rotation manner in a vessel (e.g. flask, plate) comprising a conventional cell culture medium containing or not serum using a $CO_2$ incubator under 2 to 10 % $CO_2$ at 32 to 37° C. The conventional culture medium may be, for instance, a medium (e.g. RPMI1640, Eagle's MEM) containing 2 to 20 % serum of bovine fetus, calf, cow, horse, human or the like, a serum-free medium containing trace components required for the growth of cells (e.g. insulin, transferrin, ethanolamine, selenite, bovine albumin, lipid) or the like. Particularly when culture is made at a large scale, a jar fermenter, a hollow fiber system or the like designed for animal cells may be used.

Stage (4):-

Purification of the antibody may be carried out by conventional biochemical procedures such as ammonium sulfate precipitation, ethanol precipitation, PEG fractionation, ion exchange chromatography, gel filtration, affinity chromatography, high performance liquid chromatography, electrophoresis, etc. In the purification process, care should be taken for preventing the production of agglutination or the depression of antibody activity. For this purpose, human serum albumin (HSA) may be added in an amount of 0.05 to 2 %. Also, addition of amino acids such as glycine or alpha- alanine, especially basic amino acids such as lysine, arginine or histidine, saccharides such as glucose or mannitol, salts such as sodium chloride, etc. is occasionally preferred. Agglutination tends to be produced, particularly in the case of IgM antibody, and treatment with beta-propionolactone, acetic anhydride or the like is effective in prevention of such agglutination. In such case, intravenous administration without any agglutination will be made possible.

Stage (5):-

The purified monoclonal antibody may be formulated into a biological preparation by a per se conventional procedure comprising, for instance, filtering through a membrane filter for removal of bacteria and admitting into sterilized vials with a stabilzer, followed by lyophilyzation.

The human monoclonal antibody preparation thus prepared may comprise only one kind of human monoclonal antibody reactive to one antigenic determinant of flagellin of P. aeruginosa for its use as a prophylactic or therapeutic agent for infections caused by P. aeruginosa. Preferably, however, it comprises two or more kinds of human monoclonal antibodies which can recognize different antigenic determinants of flagellin in P. aeruginosa. It may also comprise additionally a human monoclonal antibody which can recognize any of other surface layer antigens of P. aeruginosa such as outer membrane proteins and O-antigens in LPS, pathogenic factors of P. aeruginosa such as exotoxins, exoenzymes such as elastase and protease, etc. or be admixed with any conventional human immunoglobulin preparation for them. It may be further admixed with any human antibody to bacteria other than P. aeruginosa, virus, fungi, protozoa, tumor cells, etc. or any conventional human immunoglobulin preparation for them. Incorporation of the human monoclonal antibody of the invention into a conventional human immunoglobulin preparation makes a high titer immunoglobulin preparation to P. aeruginosa. For mixed bacterial infections, the human monoclonal antibody of the invention may be applied in combination with any conventional antibiotic or synthetic antimicribial agent.

The human monoclonal antibody of the invention recognizes specifically flagellin and binds to flagella of P. aeruginosa, whereby the bacterial body is opsonized and the phagocytosis and bacteriolysis actions of phagocytes thereon are enhanced. Also, complements are activated to accelerate the lysis of the bacterial body. Further, the motility of P. aeruginosa is suppressed to lower the pathogenicity of the bacterium. Due to these actions, the human monoclonal antibody of the invention produces a significant therapeutic effect in the experimental mouse infections with P. aeruginosa even at such a small dose as 5 μg/kg. In view of this experimental result, the human monoclonal antibody of the invention may be administered to an adult human patient in an amount of about 0.05 to 1 mg per kg of body weight for the therapeutic purpose of infectious diseases with P. aeruginosa alone or mixed bacterial infection 20containing P. aeruginosa or in an amount of about 0.005 to 0.1 mg per kg of body weight for the prophylactic purpose.

As stated above, the human monoclonal antibody has a specific binding property to flagella of P. aeruginosa and exerts its therapeutic effect on the experimental mouse infections caused not only by various strains having O-antigen but also by strains having no O-antigen (Type M). This is quite noticeable, because any monoclonal antibody to flagella having material therapeutic effect on infections caused by Type M strains is never reported and of course monoclonal antibody to O-antigen has no effect. This is also noticeable, because Type M strains are isolated as the infection-causing organism not only from ordinary infectious diseases caused by P. aeruginosa with a frequency of several percent but also from cystic fibrosis and DPB known as quite serious diseases with a significantly high frequency.

In addition, it may be noted that the therapeutic effect of the human monoclonal antibody of the invention can be enhanced prominently when this is used in combination with conventional antibiotics or synthetic antimicrobial agents sensitive to P. aeruginosa, and this enhancement is significantly synergistic. This prominent synergistic effect is presumed to arise from the mechanism that the human monoclonal antibody not only opsonizes the bacterium to induce phagocytosis but also suppresses the motility of bacterium to make P. aeruginosa less virulent. This may be considered to be a characteristic feature inherent to the human monoclonal antibody according to the invention which specifically binds to flagella of P. aeruginosa.

Currently, most of serious P. aeruginosa infectious diseases are provoked by mixed bacterial infections containing P. aeruginosa. Therefore, theraphy of those diseases includes the application of conventional chemotherapeutic agents such as antibiotics and synthetic antimicrobial agents in combination with human immunoglobulin preparations comprising the human monoclonal antibody. Since enhancement of the synergistic effect in the combined use of the human monoclonal antibody with the conventional chemoterapeutic agents is prominent as noted above, it is quite advantageous for prevention and treatment of serious P. aeruginosa infectious diseases caused by mixed bacteria including P. aeruginosa.

As understood from the above, the human monoclonal antibody of the invention has various meritorious advantages. First, it binds specifically to flagella of P. aeruginosa and shows an excellent therapeutic effect in the experimental mouse infections at such a small dose of not less than 5 μg/kg of body weight. Since the effective dose for prevention or treatment of the infectious diseases is small, the immunoglobulin prepration comprising the human monoclonal antibody can be supplied with a low cost. Second, the human monoclonal antiboby shows its binding property to a wide variety of P. aeruginosa strains including Type M strains, and therefore its therapeutic effect is exerted on the infectious diseases caused by P. aeruginosa strains of different serotypes. Thus, it is possible to avoid any inconvenient operation for mixing a plurality of monoclonal antibodies, yet achieving a therapeutic effect on the infectious diseases caused by different serotypes of strains. Third, the human monoclonal antibody of the invention is a human-origin protein so that any side effect (e.g. anaphylaxis) as seen in the administration of a heterogenic protein is hardly produced. Since it is originated from a certain specific cell line, a possibility of contamination with unknown biohazardous materials is much less in comparison with conventional immunoglobulins prepared from a human blood originated from a number of persons.

The antibody-producing cell line of the invention is characteristic in showing a high proliferation and a significant antibody productivity continuously at a large scale cultivation. The large scale cultivation is possible even in a serum-free medium and stably affords the antibody in a large amount. For instance, the large scale cultivation with the human-mouse heterohybridoma cell line IN-2A8 in a 6 liter-volume jar fermenter shows good proliferation with high productivity of the antibody.

These good proliferation and high productivity of IN-2A8 are very important for the industrial large scale culture, so that clone IN-2A8 is a very useful cell line. Characteristically, the binding property of the IgM antibody produced from said cell line IN-2A8 to flagella of P. aeruginosa is varied with the $Ca^{++}$ ion concentration, the maximum binding property being in a concentration of 100 μM and the minimum (i.e. almost no) binding property being in a concentration of 1 μM or less.

According to the present invention, the human monoclonal antibody is thus artificially and stably produced with a high antibody titer in a large amount. The production process is more advantageous than conventional production processes using human blood with respect to quality control.

The present invention will be hereinafter explained in details by way of examples, but it should be understood that this invention is not limited to those examples.

Example 1

Establishment of human anti-flagellin monoclonal antibody-producing cell lines by human-mouse cell fusion:-

1) Preparation, cultivation and activation of human B lymphocytes

Peripheral blood (100 ml) having a high antibody titer to P. aeruginosa (formalin-fixed cells) in serum was taken from a healthy volunteer (donor, IN). To a centrifuge tube (50 ml-volume, Sumitomo Bakelite), there was admitted Mono-Poly Resolving Medium® (15 ml, Flow Lab.), and peripheral blood (20 ml) was slowly overlaid thereon, followed by centrifugation with a low speed centrifuge (BS-20BH, Tommy Precision Ind.) at 2,500 rpm (Roter-TS-7) and at room temperature for 15 minutes, whereby erythrocytes and lymphocytes were separated.

The portion containing lymphocytes was collected and washed three times with a Dulbecco's modified Eagle's minimum essential medium (Nisshi Pharmaceutical, hereinafter referred to as "D-MEM"), followed by calculation of the cell numbers to obtain lymphocyte cells of $1.2 \times 10^8$.

The lymphocyte cells ($1.2 \times 10^8$) were suspended in a lymphocyte-culturing medium (60 ml) containing formalin-fixed cells of P. aeruginosa, and the suspension was dispensed in 24 well microplates (Costar, #3424) at a rate of $2 \times 10^6$ lymphocyte cells/well and cultured at 37°C under 5 % $CO_2$ for 6 days. As the lymphocyte-culturing medium, there was used an RPMI-1640 medium (Nissui Pharmaceutical) containing inactivated fetal calf serum (FCS) (20 % (v/v)), sodium pyruvate (0.05 mg/ml), 2-mercaptoethanol ($5 \times 10^{-5}$ M), N-(2-hydroxyethyl)piperazin-N'-2-ethanesulfonic acid (hereinafter referred to as "HEPES") (20 mM) and plant lectin derived from pokeweed (PWM, Gibco Lab.) (0.01 % (v/v)).

(2) Cell fusion

Mouse myeloma cells P3x63-Ag8.653 (ATCC No. CRL1580; J. Immunol., 123, 1548 (1979)) were subcultured in D-MEM containing 10 % FCS, and $4 \times 10^7$ cells were washed twice with D-MEM.

Separately, peripheral blood lymphocytes were cultured for 6 days in 24 well microplates according to Example 1-(1) and recovered to give $8 \times 10^7$ lymphocyte cells. The cells were washed with D-MEM three times and mixed with the above mouse myeloma cells in a centrifuge tube, followed by centrifugation. To the precipitate in the centrifuge tube while rotation, there was added a polyethyleneglycol (PEG) solution (1 ml) containing PEG4000 (Merck) (0.45 g), PBS(-) (0.45 ml) and dimethylsulfoxide (0.1 ml) in about one minute, and the mixture was allowed to stand at room temperature for one minute. D-MEM was then added to the tube at a rate of 2 ml/minute while rotation, which was repeated seven times. Finally, 2 ml of FCS was added to the tube, and the mixture was allowed to stand for 20 minutes at 37°C. The cells were collected by centrifugation and suspended in 50 ml of a D-MEM medium containing FCS (15 %), sodium pyruvate (0.05 mg/ml), insulin (0.2 U/ml), oxaloacetic acid (0.15 mg/ml), azaserine (100 μM) and hypoxanthine (100 μM) (hereinafter referred to as "HAz selection medium"). The suspension was dispensed in 96 well microplates (#25860 MP, Corning Glass Works) at a rate of $8 \times 10^4$ myeloma cells/well. Simultaneously, each of the microplates was charged with mouse BALB/c spleen cells ($1 \times 10^5$) and mouse BALB/c peritoneal exudate cells ($6.5 \times 10^4$) as the feeder layer, and incubation was made at 37°C under 5 % $CO_2$. The half of the culture medium was replaced by the HAz selection medium every 2 or 3 days. After one week, the half of the culture medium was replaced by an H-medium which corresponds to the HAz selective medium but excluding azaserine therefrom. Thereafter, the half of the culture medium was replaced by an azaserine and hypoxanthine-free hybridoma-culturing D-MEM medium containing FCS (15 %), sodium pyruvate (0.05 mg/ml), insulin (0.2 U/ml) and oxaloacetic acid (0.15 mg/ml) every 2 or 3 days.

About 3 weeks after the cell fusion, production of the antibody to the antigen at the surface layer of P. aeruginosa surface was determined on the supernatants of the wells where proliferation was observed, by enzyme linked immuno sorbent assay (ELISA) using 96 well microplates (#3912F, Bector Dickinson Lab.) on which P. aeruginosa was fixed by glutaraldehyde. In two wells, production of the IgM antibody showing a strong binding property to P. aeruginosa IID1002 (the standard strain of Type 2 according to Homma et al. classification, obtainable from ATCC or from the Institute of Medical Science, Tokyo University, Japan) was confirmed. The heterohybridoma in these wells was further cultivated in 96 well microplates and cloned by the limiting dilution method under the condition giving a uniform cell in each well to obtain heterohybridoma cell lines IN-2A8 and IN-5D6 respectively having stable productivity of human monoclonal antibodies IN-2A8 and IN-5D6. Hybridoma IN-2A8 was deposited under an accession No. FERM P-9960 on March 24, 1988 at Fermentation Research Institute, Agency of Industrial Science and Technology, located at Tsukuba, Ibaraki-ken, Japan, and this deposition was converted into an internatinal deposition under Budapest Treaty on January 22, 1990 under an accession No. BP-2741.

Example 2

Establishment of human anti-flagellin monoclonal antibody-producing cell lines by human-mouse cell fusion:-

Peripheral blood (100 ml) having a high antibody titer to P. aeruginosa (formalin-fixed cells) in serum was taken from a healthy volunteer (donor, ZI), and preparation, cultivation and activation of human B lymphocytes as well as cell fusion were carried out in the same manner as in Example 1 to give a human-mouse heterohybridoma cell line ZI-3A8 capable of producing the IgM antibody showing a strong binding property to P. aeruginosa IID1002, i.e. a human monoclonal antibody designated as "ZI-3AB".

Example 3

Binding property of human IgM monoclonal antibodies IN-2A8, IN-5D6 and ZI-3A8 to standard strains of P. aeruginosa:-

The binding properties of the antibodies IN-2A8, IN-5D6 and ZI-3A8 to the standard strains of P. aeruginosa according to the Homma et al classification (obtained from the Institute of Medical Science, Tokyo University, Japan) were examined by ELISA, and the results are shown in Table 2, from which it is uderstood that the antibodies IN-2A8, IN-5D6 and ZI-3A8 show a strong binding property to IID1002 (Type 2), IID1009 (Type 9), IID5141 (Type 14), IID5004 (Type 16) and IID1015 (Type 17), and their binding spectra are quite similar to each other.

Table 2:

| Binding property of antibodies IN-2A8, IN-5D6 and ZI-3A8 to standard strains of P . aeruginosa | | | | | |
|---|---|---|---|---|---|
| Strains | Homma et al Classification | Japanese Commitee | ELISA value ($OD_{405}$) | | |
| | | | IN-2A8 | IN-5D6 | ZI-3A8 |
| IID1001 | 1 | A | 0.19 | 0.03 | 0.04 |
| IID1002 | 2 | B | 0.97 | 1.99 | 1.60 |
| IID1021 | 3 | C | 0.20 | 0.04 | 0.01 |
| IID1004 | 4 | D | 0.16 | 0.00 | 0.00 |
| IID1130 | 5 | E | 0.18 | 0.00 | 0.02 |
| IID1006 | 6 | F | 0.17 | 0.00 | 0.00 |
| IID1007 | 7 | B | 0.20 | 0.06 | 0.01 |
| IID1020 | 8 | G | 0.16 | 0.00 | 0.00 |
| IID1009 | 9 | H | 2.07 | 2.23 | 2.29 |
| IID1010 | 10 | I | 0.04 | 0.00 | 0.00 |
| IID1011 | 11 | J | 0.05 | 0.00 | 0.03 |
| IID1012 | 12 | K | 0.04 | 0.00 | 0.00 |
| IID1013 | 13 | B | 0.20 | 0.02 | 0.01 |
| IID5141 | 14 | L | 0.53 | 0.45 | 0.52 |
| IID5018 | 15 | M | 0.05 | 0.00 | 0.00 |
| IID5004 | 16 | B | 0.76 | 1.75 | 1.54 |
| IID1015 | 17 | M | 0.57 | 0.80 | 0.73 |
| (-) | | | 0.04 | 0.00 | 0.05 |

## Example 4

Binding property of antibidies IN-2A8, IN-5D6 and ZI-3A8 to clinical isolates of P. aeruginosa:-

The binding properties of the antibodies IN-2A8, IN-5D6 and ZI-3A8 to P. aeruginosa clinical isolates Types A, B, E, G, H, I and M (according to Japanese Commitee classification) were examined by ELISA, and the results are shown in Table 3, from which it is understood that the antibodies IN-2A8, IN-5D6 and ZI-3A8 show binding properties to 58 % of Type A, 55 % of Type B, 30 % of Type I, 46 % of Type H and 35 % of Type M and no binding property to Types E and G, and their binding spectra are quite similar to each other. Thus, these antibodies may be considered to re cognize identicial antigens, which have certain correlations to the serotype classification on O-antigens but are not exactly identical therewith.

Table 3:  Binding property of antibodies IN-2A8,
IN-5D6 and ZI-3A8 to P. aeruginosa
clinical isolates

| Strains | Serotype | ELISA value ($OD_{405}$) | | |
|---------|----------|---------|---------|---------|
| | | IN-2A8 | IN-5D6 | ZI-3A8 |
| SP 6745 | A | 0.08 | 0.05 | 0.05 |
| SP 6746 | A | 1.70 | 1.33 | 1.77 |
| SP 6783 | A | 0.05 | 0.05 | 0.07 |
| SP 6818 | A | 1.93 | 1.56 | 1.94 |
| SP 6830 | A | 1.12 | 0.82 | 1.29 |
| SP 6840 | A | 0.12 | 0.06 | 0.05 |
| SP 9708a | A | 1.54 | 1.22 | 1.62 |
| SP 9710 | A | 1.98 | 1.72 | 1.95 |
| SP 9711 | A | 1.47 | 1.31 | 1.80 |
| SP 9731 | A | 0.07 | 0.04 | 0.05 |
| SP 9762 | A | 0.07 | 0.03 | 0.10 |
| SP 9763 | A | 0.06 | 0.05 | 0.05 |
| SP 9768 | A | 1.59 | 1.25 | 1.94 |
| SP 9780 | A | 1.83 | 1.46 | 2.90 |
| SP 10029 | A | 1.50 | 1.09 | 1.72 |
| SP 10040 | A | 0.41 | 0.36 | 0.51 |
| SP 10060 | A | 0.71 | 0.54 | 0.96 |
| SP 10648 | A | 0.03 | 0.02 | 0.23 |
| SP 10676 | A | 0.05 | 0.05 | 0.04 |
| SP 9703 | B | 0.07 | 0.04 | 0.04 |
| SP 9722 | B | 1.73 | 2.21 | 0.04 |
| SP 9737a | B | 1.69 | 1.87 | 2.55 |
| SP 9756 | B | 0.02 | 0.07 | 0.05 |
| SP 9770 | B | 0.03 | 0.03 | 0.05 |
| SP 9791 | B | 0.05 | 0.05 | 0.08 |
| SP 10036 | B | 1.27 | 1.48 | 2.27 |
| SP 10052 | B | 1.80 | 1.83 | 2.58 |
| SP 10065 | B | 0.76 | 0.71 | 1.01 |
| SP 10069 | B | 1.02 | 0.90 | 1.38 |
| SP 10650 | B | 0.03 | 0.03 | 0.02 |
| SP 10663 | B | 0.03 | 0.03 | 0.03 |
| SP 10658 | B | 0.06 | 0.04 | 0.02 |
| SP 10680 | B | 0.08 | 0.05 | 0.07 |
| SP 6897 | B | 1.76 | 1.80 | 2.39 |
| PAO 1 | B | 2.03 | 1.98 | 2.31 |
| M 2 | B | 2.03 | 1.73 | 2.52 |
| TL 2423 | B | 0.02 | 0.03 | 0.07 |
| TL 2460 | B | 0.17 | 0.14 | 0.16 |
| SP 6747 | B | 1.60 | 1.56 | 2.36 |

(Continued)

| | | | | |
|---|---|---|---|---|
| SP 9702 | E | 0.06 | 0.06 | 0.00 |
| SP 9715 | E | 0.10 | 0.10 | 0.00 |
| SP 9720 | E | 0.07 | 0.07 | 0.00 |
| SP 9723 | E | 0.10 | 0.10 | 0.08 |
| SP 9726 | E | 0.06 | 0.06 | 0.00 |
| SP 9733 | E | 0.05 | 0.05 | 0.01 |
| SP 9740 | E | 0.05 | 0.05 | 0.00 |
| SP 9754 | E | 0.07 | 0.07 | 0.01 |
| SP 9759 | E | 0.07 | 0.05 | 0.03 |
| SP 9771 | E | 0.05 | 0.05 | 0.00 |
| SP 9783 | E | 0.06 | 0.05 | 0.00 |
| SP 9787 | E | 0.05 | 0.05 | 0.00 |
| SP 9790 | E | 0.05 | 0.05 | 0.00 |
| SP 10043 | E | 0.05 | 0.05 | 0.00 |
| SP 10059 | E | 0.05 | 0.04 | 0.00 |
| PA 103 | E | 0.05 | 0.06 | 0.00 |
| PA 103-29 | E | 0.07 | 0.05 | 0.01 |
| NC 5 | E | 0.06 | 0.04 | 0.00 |
| TL 2092 | E | 0.08 | 0.06 | 0.02 |
| TL 2158 | E | 0.06 | 0.06 | 0.02 |
| SP 9701 | G | 0.00 | 0.00 | 0.00 |
| SP 9709 | G | 0.00 | 0.00 | 0.00 |
| SP 9712 | G | 0.00 | 0.00 | 0.00 |
| SP 9714 | G | 0.00 | 0.00 | 0.00 |
| SP 9717 | G | 0.00 | 0.00 | 0.00 |
| SP 9718 | G | 0.00 | 0.00 | 0.00 |
| SP 9738 | G | 0.00 | 0.00 | 0.00 |
| SP 9785 | G | 0.00 | 0.00 | 0.00 |
| SP 9743 | G | 0.00 | 0.00 | 0.00 |
| SP 9792 | G | 0.00 | 0.00 | 0.00 |
| SP 9755 | G | 0.00 | 0.00 | 0.00 |
| SP 9761 | G | 0.00 | 0.00 | 0.00 |
| SP 9767 | G | 0.00 | 0.00 | 0.00 |
| SP 9772 | G | 0.00 | 0.00 | 0.00 |
| CTN11187 | G | 0.00 | 0.00 | 0.00 |
| TL 2378 | G | 0.00 | 0.00 | 0.00 |
| TL 2424 | G | 0.00 | 0.00 | 0.00 |
| SP 6788 | G | 0.00 | 0.00 | 0.00 |
| SP 9728a | G | 0.00 | 0.00 | 0.00 |
| SP 6896 | H | 0.61 | 1.29 | 1.30 |
| SP 6931 | H | 0.08 | 0.00 | 0.00 |
| SP 7503 | H | 0.08 | 0.00 | 0.00 |
| SP 7507 | H | 0.08 | 0.00 | 0.02 |
| SP 7514 | H | 0.08 | 0.00 | 0.01 |
| SP 7520 | H | 0.08 | 0.02 | 0.05 |
| SP 7522 | H | 1.08 | 1.97 | 1.94 |
| SP 7532 | H | 1.18 | 2.03 | 2.16 |
| SP 7555 | H | 0.08 | 0.02 | 0.01 |
| SP 10054 | H | 0.08 | 0.00 | 0.00 |
| SP 10068 | H | 0.93 | 1.92 | 1.97 |
| SP 10678 | H | 1.16 | 2.14 | 2.15 |
| SP 10681 | H | 0.45 | 0.98 | 1.20 |

(Continued)

| SP 6761 | I | 0.00 | 0.00 | 0.04 |
|---|---|---|---|---|
| SP 6722 | I | 0.00 | 0.00 | 0.04 |
| SP 6805 | I | 0.00 | 0.00 | 0.05 |
| SP 6811 | I | 0.39 | 0.36 | 0.32 |
| SP 6829 | I | 0.00 | 0.00 | 0.03 |
| SP 6839 | I | 0.27 | 0.28 | 0.31 |
| SP 6853 | I | 0.35 | 0.27 | 0.30 |
| SP 6880 | I | 0.00 | 0.00 | 0.03 |
| SP 9704 | I | 0.00 | 0.00 | 0.04 |
| SP 9705 | I | 0.00 | 0.00 | 0.03 |
| SP 9706 | I | 0.00 | 0.00 | 0.06 |
| SP 9707 | I | 0.00 | 0.00 | 0.07 |
| SP 9735 | I | 0.14 | 0.15 | 0.22 |
| SP 9757 | I | 0.00 | 0.00 | 0.03 |
| SP 9776 | I | 0.00 | 0.00 | 0.03 |
| SP 9779 | I | 0.00 | 0.00 | 0.04 |
| SP 9799 | I | 0.00 | 0.00 | 0.06 |
| SP 10041 | I | 0.00 | 0.00 | 0.05 |
| SP 10046 | I | 0.71 | 0.68 | 0.67 |
| SP 10670 | I | 0.27 | 0.26 | 0.29 |
| SP 9716 | M | 0.06 | 0.03 | 0.08 |
| SP 9730 | M | 0.73 | 0.57 | 0.81 |
| SP 9744 | M | 0.05 | 0.04 | 0.04 |
| SP 9748 | M | 1.16 | 1.00 | 1.33 |
| SP 9749 | M | 0.01 | 0.02 | 0.01 |
| SP 9752 | M | 0.11 | 0.09 | 0.10 |
| SP 9775 | M | 0.01 | 0.01 | 0.01 |
| SP 10067 | M | 0.01 | 0.01 | 0.01 |
| SP 10675 | M | 0.02 | 0.02 | 0.02 |
| SP 6763 | M | 1.66 | 1.36 | 1.61 |
| SP 6764 | M | 1.82 | 1.68 | 1.90 |
| SP 6765 | M | 1.64 | 1.46 | 1.74 |
| SP 6782 | M | 0.03 | 0.01 | 0.01 |
| SP 6794 | M | 0.01 | 0.01 | 0.01 |
| SP 6833 | M | 0.04 | 0.03 | 0.02 |
| SP 6852 | M | 1.51 | 1.55 | 1.57 |
| SP 6890 | M | 0.04 | 0.03 | 0.02 |
| SP 6892 | M | 0.82 | 0.72 | 0.94 |
| SP 6895 | M | 0.01 | 0.01 | 0.01 |
| SP 6908 | M | 0.01 | 0.01 | 0.01 |
| (-) | | 0.01 | 0.04 | 0.02 |

Example 5

Antigen to be recognized by antibody IN-2A8:-

**(1) Preparation of crude flagella product from P. aeruginosa**

The crude preparation of flagella of P. aeruginosa was prepared by the method as described by Pitt et al (J. Med. Microbiol., 14, 251 (1981)). P. aeruginosa strain M2 (Serotype B according to the Japanese Commitee classification), obtained from Shrinens Burns Institute, Cincinnati, Ohio, U.S.A. (J. Infect. Dis., 131, 688 (1975)) and clinical isolate strain SP 6818 (Serotype A), which shows a binding property to the IgM antibody produced by Hybridoma IN-2A8, IN-5D6 or ZI-3A8 in ELISA, were used.

Each of the above strains was cultured on a heart-infusion agar medium (Nissui Seiyaka) for 18 hours. The bacterial cells were scraped from the agar surface and suspended in potassium and magnesium-free, phosphate-buffered saline (hereinafter referred to as "PBS(-)") (10 ml), followed by centrifugation at 5000xg at 4°C for 15 minutes. The precipited bacterial cells were again suspended in PBS(-) (5 ml) and mixed by the aid of a Vortex mixer for 3 minutes to release flagella from the bacterial cells. Centrifugation was continued at 16000xg at 4°C for 15 minutes, and after removal of the bacterial cells, the supernatant was further centrifuged at 40000xg at 4°C for 3 hours to precipitate flagella. The precipitants were suspended in PBS(-) (400 μl) to obtain a crude flagella preparation.

**(2) Electrophoretic analysis of the crude flagella preparation on SDS-polyacrylamide gel**

The crude flagella preparation obtained in (1) and a molecular weight marker were mixed with a sample buffer (50 μl) comprising tris buffer (6.25 mM; pH 6.8), SDS (2 %), 2-mercaptoethanol (10 %), glycerol (10 %) and BPB (0.001 %), and the mixture was heated at 95°C for 5 minutes and subjected to electrophoresis on 10 % polyacrylamide gel containing 0.2 % SDS and staining with Coomassie Brilliant Blue G® (Bio-Lad.). For the crude flagella preparation originated from strain M2 or strain SP 6818, a major band was recognized at the position of about 52,000 in molecular weight, with a slight amount of impurities at the position of about 60,000 in molecular weight. The above molecular weight of about 52,000 for the crude flagella preparation is almost identical to the molecular weight of 53,000 reported for strain M2-originated flagelin by Montie et al (Infec. Immun., 35, 281 (1982)).

**(3) Binding property of the IgM antibody produced by Hybridoma IN-2A8 as examined by western blotting method**

Western blotting (Proc. Natl. Acad. Sci., U.S.A., 76, 3116 (1979)) was conducted to examine the binding property of the IgM antibody to the crude flagella preparation. Namely, the crude flagella preparation as obtained in (1) was subjected to electrophoresis in the same manner as in (2) and then electrically transferred onto a polyvinylidenedifluoride membrane (Durapore®, Japan Millipore). On the membrane, it was recognized by the enzyme immuno staining method that the human monoclonal antibody IgM produced by hybridoma IN-2A8 was bound to the major band corresponding to flagellin having a molecular weight of 52,000, as shown in (2), in the crude flagella preparation originated from strain M2 or SP 6818.

Still, the crude flagella preparation was treated with a protease (Proteinase K) at 37°C for 2 hours and then subjected to western blotting. As the result, it was confirmed that the crude flagella preparation lost the reactivity with the IgM antibody produced by Hybridoma IN-2A8.

From the above results, it is understood that the IgM antibody produced by hybridoma IN-2A8 specifically recognizes a granular protein constituting flagella of P. aeruginosa, i.e. flagellin, and is bound to the flagella. Likewise, the IgM antibodies produced from Hybridomas IN-5D6 and ZI-3A8 can be bound to flagellin.

In summary, the IgM antibody produced by Hybridoma IN-2A8, IN-5D6 or ZI-3A8 was confirmed by the western blotting method to show a binding property to the strain M2-originated flagellin. It was also confirmed by the ELISA method that said antibody shows a binding property to the strains M2 and PAO-1 (ATCC2524) of P. aeruginosa. Since both of these strains are classified into Serotype Hb according to the classification of Ansorg et al. on flagella (Allison, Infec. Immun., 49, 770 (1985)), the IgM antibodies produced from Hybridomas IN-2A8, IN-5D6 and ZI-3A8 may be considered to have a specific binding property to Serotype b flagella according to the classification of Ansorg et al. and its constituent flagellin.

Example 6

Therapeutic effect of Antibody IN-2A8 on the experimental P. aeruginosa infection in mice (1):-

ICR strain mice (4 week old; male; 10 animals per group; purchased from Experimental Animal Farm Cooperation, Shizuoka-ken, Japan) were shaved on the back, anesthetized and forced to have a burnt on the back with pressing a burnt ethanol-impregnated glass fiber thereto for 10 seconds. In order to relieve the burnt shock, physiological saline (0.3 ml) was intraperitoneally administered to each mouse, and then a physiological saline suspension of P. aeruginosa M2 strain (O-antigen serotype B) or clinical isolate strain SP 10052 (O-antigen serotype B) having a designed bacterial concentration (0.2 ml) was subcutaneously administered at the burn site for infection. After 1 hour, the human monoclonal antibody IgM produced from Hybridoma IN-2A8, being diluted with PBS(-) to make a designed concentration, was intraveneously injected (0.2 ml) to each mouse. After 7 days, the survival rate was observed to determine the therapeutic effect. The results are shown in Table 4, from which it is understood that the human monoclonal antibody IgM originated from Hybridoma IN-2A8 clearly shows an increase of the survival rate either at a dose of 0.1 $\mu$g/head or 1 $\mu$g/head in comparisown with the non-medicated group.

The $LD_{50}$ value as well as its upper and lower limits with a 95 % confidence limit for each dose were statistically calculated from the survival rate as shown in Table 4, and the results are shown in Table 5, from which it is understood that Antibody IN-2A8 shows a high $LD_{50}$ value either at a dose of 0.1 $\mu$g/head or 1 $\mu$g/head in comparison wit the non-medicated group or the BSA-treated group. It is also understood that the range between the upper and lower limits of the $LD_{50}$ value with the 95 % confidence limit for the medicated group was not overlapped with that for the non-medicated group or that for the BSA-treated group, and the difference is thus significant. In other words, Antibody IN-2A8 may be understood to exert a definite therapeutic effect either at a dose of 0.1 $\mu$g/head or 1 $\mu$g/head.

Table 4-1: Therapeutic effect of Antibody IN-2A8 on experimental infection caused by M2 strain of P. aeruginosa in mice

| Antibody | Dose (µg/head) | Survival rate (%)[1] (Inoculated amount: CFU/head) | | | |
|---|---|---|---|---|---|
| | | $1.3 \times 10^3$ | $1.3 \times 10^4$ | $1.3 \times 10^5$ | $1.3 \times 10^6$ |
| IN-2A8 | 0.1 | 100 | 90 | 90 | 20 |
| BSA[2] | 0.1 | 60 | 50 | 20 | 0 |
| Non-medicated | - | 30 | 40 | 10 | 0 |

Note: [1] Survival rate on groups each consisting of 10 animals
[2] bovine serum albumin

Table 4-2: Therapeutic effect of Antibody IN-2A8 on experimental infection caused by clinical isolate strain (SP 10052) of P. aeruginosa in mice

| Dose of IN-2A8 (µg/head) | Survival rate (%)[3] (Inoculated amount: CFU/head) | | | | | | |
|---|---|---|---|---|---|---|---|
| | $5.7 \times 10$ | $5.7 \times 10^2$ | $5.7 \times 10^3$ | $5.7 \times 10^4$ | $2.3 \times 10^5$ | $1.1 \times 10^6$ | $5.7 \times 10^6$ |
| 0.01 | N.D.[4] | 56 | 33 | 22 | 0 | N.D. | N.D. |
| 0.1 | N.D. | N.D. | 89 | 56 | 44 | 11 | N.D. |
| 1 | N.D. | N.D. | N.D. | 100 | 100 | 67 | 33 |
| Non-medicated | 56 | 44 | 44 | 22 | N.D. | N.D. | N.D. |

Note: *3) Survival rate on groups each consisting of 9 animals
*4) N.D., not examined

Table 5-1:

| LD$_{50}$ value in experimental infection caused by M2 strain of P . aeruginosa in mice | | |
|---|---|---|
| Antibody | Dose (μg/head) | LD$_{50}$ value (95 % confidence limit) |
| IN-2A8 | 0.1 | $4.3 \times 10^5$ ($1.3 \times 10^5$ - $14 \times 10^{5)}$ |
| BSA | 0.1 | $5.7 \times 103$ ($1.2 \times 10^3$ - $29 \times 10^{3)}$ |
| Non-medicated | - | $<1.3 \times 10^3$ |

Table 5-2:

| LD$_{50}$ value in experimental infection caused by clinical isolate (SP 10052) of P . aeruginosa in mice | |
|---|---|
| Dose of IN-2A8 ($\mu$g/head) | LD$_{50}$ value (95 % confidence limit) |
| 0.01 | $1.2 \times 10^3$ ($1.3 \times 10^2$ - $1.1 \times 10^{4)}$ |
| 0.1 | $1.0 \times 10^5$ ($2.2 \times 10^4$ - $4.5 \times 10^{5)}$ |
| 1 | $2.8 \times 10^6$ ($1.2 \times 10^6$ - $6.5 \times 10^6$) |
| Non-medicated | $2.6 \times 10^2$ ($4.9$ - $1.5 \times 10^4$) |

Example 7

Therapeutic effect of Antibody IN-2A8 on experimental P. aeruginosa infection in mice (2):-

In the same manner as in Example 6 but using different serotype strains of P. aeruginosa, the therapeutic effect of Antibody IN-2A8 was examined through the survival rate and the $LD_{50}$ value at a dose of 1 $\mu$g/head as shown in Table 6.

Table 6-1: Therapeutic effect of Antibody IN-2A8 on experimental infection caused by clinical isolate (SP 6818, Type A according to O-antigen serotype classification) of $\underline{P}$. $\underline{aeruginosa}$ in mice

| Antibody | Dose (µg/head) | Survival rate (%)[1] (Inoculated amount: CFU/head) | | | | |
|---|---|---|---|---|---|---|
| | | $3.3 \times 10^4$ | $1.3 \times 10^5$ | $6.5 \times 10^5$ | $3.3 \times 10^6$ | $1.3 \times 10^7$ |
| IN-2A8 | 1 | N.D.[2] | 100 | 80 | 80 | 0 |
| Non-medicated | - | 80 | 100 | 40 | 10 | N.D. |

Note: *1) Survival rate on groups each consisting of 10 animals
*2) N.D., not examined

Table 6-1:

| LD$_{50}$ value | |
| --- | --- |
| Dose ($\mu$g/head) | LD$_{50}$ value (95 % confidence limit) |
| 1 | $3.4 \times 10^6$ ($1.2 \times 10^6$ - $9.6 \times 10^6$) |
| Non-medicated | $3.8 \times 10^5$ ($1.5 \times 10^5$ - $1 \times 10^6$) |

Table 6-3: Therapeutic effect of Antibody IN-2A8 on experimental infection caused by clinical isolates (SP 6830, Type A strain according to O-antigen serotype classification) of P. aeruginosa in mice

| Antibody | Dose (μg/head) | Survival rate (%)[*1] (Inoculated amount: CFU/head) | | | | |
|---|---|---|---|---|---|---|
| | | $2.6 \times 10^5$ | $1.3 \times 10^5$ | $5.2 \times 10^6$ | $2.6 \times 10^7$ | $1.3 \times 10^8$ |
| IN-2A8 | 1 | N.D. | 100 | 100 | 50 | 0 |
| Non-medicated | | 100 | 80 | 50 | 0 | N.D. |

Note:  *1)  Survival rate on groups each consisting of 10 animals

Table 6-4:

| LD$_{50}$ value | |
|---|---|
| Dose (µg/head) | LD$_{50}$ value (95 % confidence limit) |
| 1 | $2.6 \times 10^7$ ($1.7 \times 10^7$ - $4.1 \times 10^7$) |
| Non-medicated | $1.5 \times 10^6$ ($5 \times 10^5$ - $4.3 \times 10^5$) |

Table 6-5: Therapeutic effect of Antibody IN-2A8 on experimental infection caused by clinical isolate (SP 6897, Type B according to O-antigen serotype classification) of P. aeruginosa in mice

| Antibody | Dose (µg/ head) | Survival rate (%)[1] (Inoculated amount: CFU/head) | | | | | | |
|---|---|---|---|---|---|---|---|---|
| | | $3.1 \times 10$ | $3.1 \times 10^2$ | $3.1 \times 10^3$ | $3.1 \times 10^4$ | $1.5 \times 10^5$ | $6.1 \times 10^5$ | $3.1 \times 10^6$ |
| IN-2A8 | 1 | N.D. | N.D. | N.D. | 90 | 100 | 50 | 20 |
| Non-medicated | - | 40 | 40 | 20 | 10 | N.D. | N.D. | N.D. |

Note: *1) Survival rate on groups each consisting of 10 animals

Table 6-6:

| LD$_{50}$ value | |
|---|---|
| Dose ($\mu$g/head) | LD$_{50}$ value (95 % confidence limit) |
| 1 | $8.2 \times 10^5$ ($3.4 \times 10^5$ - $2.0 \times 10^6$) |
| Non-medicated | $1.5 \times 10$ (0.56 - $3.9 \times 10^2$) |

EP 0 383 090 A1

Table 6-7: Therapeutic effect of Antibody IN-2A8 on experimental infection caused by clinical isolate (SP 9737a, Type B according to O-antigen serotype classification) of P. aeruginosa in mice

| Antibody | Dose (µg/head) | Survival rate (%)[*1] (Inoculated amount: CFU/head) | | | | | | |
|---|---|---|---|---|---|---|---|---|
| | | $2.2 \times 10^4$ | $2.2 \times 10^5$ | $2.2 \times 10^6$ | $1.1 \times 10^7$ | $2.2 \times 10^7$ | $4.5 \times 10^7$ | $2.2 \times 10^8$ |
| IN-2A8 | 1 | N.D. | N.D. | 100 | 90 | N.D. | 20 | 0 |
| Non-medicated | - | 100 | 90 | 20 | N.D. | 0 | N.D. | N.D. |

Note: *1) Survival rate in one group consisting of 10 animals

Table 6-8:

| LD$_{50}$ value | |
|---|---|
| Dose ($\mu$g/head) | LD$_{50}$ value (95 % confidence limit) |
| 1 | $2.6 \times 10^7$ ($1.5 \times 10^7$ - $4.5 \times 10^7$) |
| Non-medicated | $8.9 \times 10^5$ ($3.5 \times 10^5$ - $2.3 \times 10^6$) |

Table 6-9: Therapeutic effect of Antibody IN-2A8 on experimental infec-
tion caused by clinical isolate (SP 6853, Type I according to
O-antigen serotype classification) of P. aeruginosa in mice

| Antibody | Dose (µg/head) | Survival rate (%)[1] (Inoculated amount: CFU/head) | | | | |
|---|---|---|---|---|---|---|
| | | $3.0 \times 10^4$ | $3.0 \times 10^5$ | $1.5 \times 10^6$ | $7.6 \times 10^6$ | $3.0 \times 10^7$ |
| IN-2A8 | 1 | N.D. | 100 | 50 | 0 | 0 |
| Non-medicated | - | 70 | 40 | 0 | 0 | N.D. |

Note: *1) Survival rate on groups each consisting of 10 animals

Table 6-10:

| LD$_{50}$ value | |
| --- | --- |
| Dose ($\mu$g/head) | LD$_{50}$ value (95 % confidence limit) |
| 1 | $1.5 \times 10^6$ ($9.6 \times 10^5$ - $3.6 \times 10^6$) |
| Non-medicated | $1.0 \times 10^5$ ($3.5 \times 10^4$ - $2.9 \times 10^5$) |

Table 6-11: Therapeutic effect of Antibody IN-2A8 on experimental infection caused by clinical isolate (SP 10046, Type I according to O-antigen serotype classification) of $\underline{P.}$ $\underline{aeruginosa}$ in mice

| Antibody | Dose (μg/head) | Survival rate (%)[1] (Inoculated amount: CFU/head) | | | | |
|---|---|---|---|---|---|---|
| | | $2.6 \times 10^4$ | $1.3 \times 10^5$ | $5.2 \times 10^6$ | $2.6 \times 10^6$ | $1.3 \times 10^7$ |
| IN-2A8 | 1 | N.D. | 100 | 90 | 60 | 0 |
| Non-medicated | - | 100 | 80 | 50 | 0 | N.D. |

Note: *1) Survival rate on groups each consisting of 10 animals

Table 6-12:

| LD$_{50}$ value | |
| --- | --- |
| Dose ($\mu$g/head) | LD$_{50}$ value (95 % confidence limit) |
| 1 | $2.5 \times 10^6$ ($1.1 \times 10^6$ - $5.7 \times 10^6$) |
| Non-medicated | $3.9 \times 10^5$ ($1.6 \times 10^5$ - $9.6 \times 10^5$) |

As understood from the above results, Antibody IN-2A8 shows a therapeutic effect on the infection caused by various O-antigen serotype strains of P. aeruginosa. Further, the range between the upper and lower limits of the LD$_{50}$ value with the 95 % confidence limit is not overlapped with that of the non-medicated group, and the difference is thus significant. It follows therefrom that the treatment with Antibody IN-2A8 at a dose of 1 $\mu$g/head has a significant therapeutic effect.

Example 8

Large scale cultivation of Hybridoma IN-2A8 with jar fermenter:-

Hybridoma IN-2A8 was cultured in a jar fermenter (6 liter-volume, Model MCT-3S, Marubishi Bioengineering) using a commercially available serum-free culture medium (Cellgrowther H®, Meguro Lab.) (4.4 liters) containing glucose (final concentration, 4 mg/ml) and FCS (2 %). Hybridoma IN-2A8 was inoculated therein at a concentration of $1 \times 10^5$ cells/ml and cultivated for 3 days to make a concentration of $4.1 \times 10^5$ cells/ml. The concentration of the human monoclonal antibody in the supernatant reached to 5.3 $\mu$g/ml. Every 2 to 3 days, stirring was intermittently stopped to precipitate the cells, followed by replacement of the upper supernatant containing no cell with a fresh medium. With continuous cultivation in this way, a high concentration of more than $1 \times 10^6$ cells/ml could be maintained, and the concentration of the human monoclonal antibody in the supernatant reached to 10 $\mu$g/ml at that stage.

Example 9

Purification of Antibody IN-2A8:-

In the same manner as in Examle 8, large scale cultivation of Hybridoma IN-2A8 was performed in a jar fermenter (6 liter-volume) using the Cellgrowther H® medium containing FCS (3 %). The supernatant (5.5 l) was ultra-condensed to 100 ml (protein content, 2.6 g) and charged on an Q-sepharose FF (Pharmacia, 2.6 cm (diameter) x 30 cm (length), 150 ml volume) equilibrated with PBS(-), followed by washing with five times volume of 0.15 M phosphate-buffer (pH, 6.8) and eluting with 0.3 M phosphate-buffer to obtain IgM fractions. The fractions were subjected to ultracondensation and charged on sephacryl S300HR® (Pharmacia, 2.6 cm (diameter) x 90 cm (length), 450 ml volume), followed by elution with PBS(-). The anti-flagella antibody titer of each fraction was determined on ELISA, and the active fractions were recovered. The recovery rate of the human monoclonal IgM antibody as protein was 1.2 %, and its specific activity raised to about 60 times.

Example 10

Therapeutic effect of Antibody IN-2A8 on experimental P. aeruginosa infection in mice (3):-

In the same manner as in Example 6, the survival rate of mice and the LD$_{50}$ value were observed for evaluation of the therapeutic effect of Antibody IN-2A8 at a dose of 1 $\mu$g/head against Serotype M strains of P. aeruginosa chal lenge. In this example, however, an immunosuppressant (i.e. cyclophosphamide, Shionogi & Co., Ltd.) was intraperitoneally administered to the mice at a dose of 250 mg/kg 4 days before the burning and the bacterial infection so as to induce the decrease of granulocytes. The results are shown in Table 7.

Table 7-1: Therapeutic effect of Antibody IN-2A8 on experimental infection caused by clinical isolate (SP 6764, Type M according to O-antigen serotype classification) of P. aeruginosa in mice

(Inoculated amount: CFU/head)

| Antibody | Dose (μg/head) | Survival rate (%) [1] | | | |
|---|---|---|---|---|---|
| | | $2.5 \times 10^2$ | $1.2 \times 10^3$ | $5.0 \times 10^3$ | $2.5 \times 10^4$ |
| IN-2A8 | 1 | 100 | 90 | 60 | 40 |
| Non-medicated | — | 22 | 40 | 33 | 0 |

Note: *1) Survival rate on groups each consisting of 9 or 10 animals

Table 7-1:

| LD$_{50}$ value | |
| --- | --- |
| Dose ($\mu$g/head) | LD$_{50}$ value (95 % confidence limit) |
| 1 | $9.3 \times 10^4$ ($3.5 \times 10^4$ - $2.5 \times 10^5$) |
| Non-medicated | $2.6 \times 10$ ($1.4$ - $4.8 \times 10^2$) |

As understood from Table 7, Antibody IN-2A8 was confirmed to be effective on Serotype M strain of P. aeruginosa in the experimental infection on neutropenic burned mice. Further, the range between the upper and lower limits of the LD$_{50}$ values with the 95 % confidence limit is not overlapped with that of the non-mediated group, and the difference is thus significant. It follows therefrom that the treatment with the IgM antibody has a distinct therapeutic effect.

Example 11

Therapeutic effect of Antibody IN-2A8 on experimental P. aeruginosa infection in mice (4):-

The therapeutic ffect of Antibody IN-2A8 on the associated use with Antibiotic Imipenem ("Thienam"®, Banyu Pharmaceutical) was examined. In the same manner as in Example 6, the survival rate of mice and the LD$_{50}$ value were observed for evaluation of the therapeutic effect of Antibody IN-2A8. In this example, however, the mice was intraveneously administered the antibody as well as the antibiotic one hour after the bacterial infection. For suppression of the inactivation in kidney and the renal toxicity, an eqivalent amount of cyrastin was incorporated into the antibiotic. The results are shown in Table 8.

Table 8-1:
Table 8-1: Therapeutic effect of Antibody IN-2A8 in association with imipenem (IMP) on experimental infection caused by clinical isolate (SP 10052, Type B according to O-antigen serotype classification) of P. aeruginosa in mice

| Sample | Dose (µg/head) | Survival rate (%)[*1] (Inoculated amount: CFU/head) | | | | |
|---|---|---|---|---|---|---|
| | | $7.3 \times 10^2$ | $7.3 \times 10^3$ | $7.3 \times 10^4$ | $7.3 \times 10^5$ | $7.3 \times 10^6$ |
| IN-2A8 + IMP | 0.1 + 100 | – | 100 | 100 | 90 | 60 |
| IN-2A8 + IMP | 0.1 + 500 | – | 100 | 100 | 100 | 100 |
| IMP | 100 | – | 80 | 70 | 40 | 0 |
| IMP | 500 | – | 80 | 90 | 80 | 20 |
| IN-2A8 | 0.1 | – | 90 | 90 | 30 | 0 |
| Non-medicated | – | 60 | 30 | 20 | 0 | – |

Note: *1) Survival rate on groups each consisting of 9 or 10 animals

36

Table 8-2:

| LD$_{50}$ value | | |
|---|---|---|
| Sample | Dose (μg/head) | LD$_{50}$ value (95 % confidence limit) |
| IN-2A8 + IMP | 0.1 + 100 | $1.1 \times 10^6$ ($2.0 \times 10^5$ - $6.6 \times 10^6$) |
| IN-2A8 + IMP | 0.1 + 500 | more than $7.3 \times 10^6$ |
| IMP | 100 | $1.7 \times 10^5$ ($3.2 \times 10^4$ - $8.8 \times 10^5$) |
| IMP | 500 | $2.0 \times 10^6$ ($2.6 \times 10^5$ - $1.5 \times 10^7$) |
| IN-2A8 | 0.1 | $2.6 \times 10^5$ ($8.3 \times 10^4$ - $8.5 \times 10^5$) |
| Non-medicated | - | $1.8 \times 10^3$ ($2.4 \times 10^2$ - $1.3 \times 10^4$) |

As apparent from the survival rate and the LD$_{50}$ values in Table 8, the associated use of Antibody IN-2A8 and Antibiotic imipenem showed a prominent therapeutic effect in comparison with their sole use. It follows therefrom that the antibody and the antibiotic work synergistically to exert their therapeutic effects..

Example 12

Influence of Ca$^{++}$ ion concentration on the binding property of Antibody IN-2A8:-

Influence of Ca$^{++}$ ion concentratin on the binding property of Antibody IN-2A8 was studied by ELISA using 96 well microplates on which the strain IDD1002 of P. aeruginosa was fixed. The results are shown in Table 9, from which it is confirmed that the Ca$^{++}$ ion concentration in the buffer solution on the progress of the antigen-antibody reaction affords a great influence on the binding property of the antibody to the flagella of P. aeruginosa.

Table 9:

| ELISA value (OD$_{405}$) | | | | |
|---|---|---|---|---|
| Ca$^{++}$ Concentration | Concentration of Antibody IN-2A8 (μg/ml) | | | |
| | 0.007 | 0.035 | 0.18 | 0.88 |
| 1 μM | 0.01 | 0.01 | 0.03 | 0.10 |
| 10 μM | 0.16 | 0.47 | 0.65 | 0.73 |
| 100 μM | 0.43 | 0.82 | 0.98 | 1.01 |
| 1 mM | 0.01 | 0.16 | 0.71 | 0.86 |
| 10 mM | 0.01 | 0.01 | 0.04 | 0.68 |

As shown in Table 9, the antibody did not exert any binding property when the Ca$^{++}$ ion concentration was less than 1 μM. It follows therefrom that the presence of Ca$^{++}$ ion is essential for the binding property between antibody and flagella. Maximum binding activity is seen in the Ca$^{++}$ ion concentraiton of around 100 μM, and the binding property rather tends to decrease when the concentration is more than 1 mM.

In the same assay as above but using Mg$^{++}$ ion, no binding property was observed in any concentration, from which it may be understood that, among divalent cations, only Ca$^{++}$ ion is essential for the binding property.

**Claims**

1. A human monoclonal antibody showing a specific binding property to flagella of Pseudomonas aeruginosa, characterized in that said antibody produces a therapeutic effect on the mouse experimental infection caused by Pseudomonas aeruginosa at a dose of not less than 5 μg/kg of body weight.

2. A human monoclonal antibody showing a specific binding property to flagella of Pseudomonsa aeruginosa, characterized in that said antibody produces a therapeutic effect on the mouse experimental infection caused by Pseudomonas aeruginosa strain having no O-antigen at the surface layer and belonging to Serotype M under the Japanese Commitee's classification.

3. The monoclonal antibody according to claim 1 or 2, which is produced from Hybridoma IN-2A8 (FERM BP-9960) or its descendant cell line.

4. A human immunoglobulin preparation which comprises an effective amount of the monoclonal antibody according to any of claims 1 to 3.

5. A prophylactic or therapeutic agent for infections with Pseudomonas aeruginosa, which comprises an effective amount of the monoclonal antibody according to any of claims 1 to 3.

6. A hybridoma or its descendant cell line capable of producing the monoclonal antibody according to any of claims 1 to 3.

7. Hybridoma IN-2A8 (FERM BP-9960) or its decendant cell line.

8. A process for producing the human monoclonal antibody according to any of claims 1 to 3, which comprises culturing the hybridoma or its descendant cell line according to any of claims 6 and 7 and recovering the produced antibody from the resultant culture.

9. The use of the monoclonal antibody according to claims 1 to 3 for the preparation of a pharmaceutical useful in treatment of bacterial infections.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 192 185 (GENETIC SYSTEMS CORP.) * Page 3, lines 32-35; page 3, lines 47-62; page 5, lines 47-61; examples 6-12; claims * --- | 1-9 | C 12 P 21/00 C 12 N 5/00 A 61 K 39/40 |
| D,Y | INFECTION AND IMMUNITY, vol. 54, no. 1, October 1986, pages 239-244, American Society for Microbiology; J.E. PENNINGTON et al.: "Polyclonal and monoclonal antibody therapy for experimental Pseudomonas aeruginosa pneumonia" * Whole article * --- | 1-9 | |
| D,Y | CAN. J. MICROBIOL., vol. 33, 1987, pages 755-763; D. DRAKE et al.: "Protection against Pseudomonas aeruginosa infection by passive transfer of anti-flagellar serum" * Whole article * ----- | 1-9 | |
| | | | TECHNICAL FIELDS SEARCHED (Int. Cl.5) C 12 P C 12 N A 61 K |

The present search report has been drawn up for all claims

| Place of search | Date of completion of the search | Examiner |
|---|---|---|
| THE HAGUE | 06-04-1990 | DIEDENHOFEN A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)